(19) Europäisches Patentamt
European Patent Office
Office européen des brevets

(11) Veröffentlichungsnummer: **0 238 576 B1**

(12) # EUROPÄISCHE PATENTSCHRIFT

(45) Veröffentlichungstag der Patentschrift: **06.05.92**

(51) Int. Cl.[5]: **C07D 239/26**, C09K 19/30, C09K 19/34, C07D 213/30, C07D 213/65, C07D 213/79, C07D 213/55

(21) Anmeldenummer: **86905780.2**

(22) Anmeldetag: **15.09.86**

(86) Internationale Anmeldenummer:
**PCT/EP86/00530**

(87) Internationale Veröffentlichungsnummer:
**WO 87/01701 (26.03.87 87/07)**

Die Akte enthält technische Angaben, die nach dem Eingang der Anmeldung eingereicht wurden und die nicht in dieser Patentschrift enthalten sind.

(54) **STICKSTOFFHALTIGE HETEROCYCLISCHE VERBINDUNGEN.**

(30) Priorität: **18.09.85 DE 3533333**

(43) Veröffentlichungstag der Anmeldung:
**30.09.87 Patentblatt 87/40**

(45) Bekanntmachung des Hinweises auf die Patenterteilung:
**06.05.92 Patentblatt 92/19**

(84) Benannte Vertragsstaaten:
**CH DE FR GB LI SE**

(56) Entgegenhaltungen:
**EP-A- 0 104 011**
**EP-A- 0 107 759**
**EP-A- 0 111 695**
**EP-A- 0 152 697**
**EP-A- 0 152 808**
**WO-A-86/07085**

(73) Patentinhaber: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **WÄCHTLER, Andreas**
**Goethestr. 34**
**W-6103 Griesheim(DE)**
Erfinder: **EIDENSCHINK, Rudolf**
**Konrad-Adenauer-Str. 1**
**W-6109 Mühltal 1(DE)**
Erfinder: **KRAUSE, Joachim**
**Samuel-Morse-Str. 14**
**W-6110 Dieburg(DE)**
Erfinder: **SCHEUBLE, Bernhard**
**Bluff 100, 100-1 Yamate-Cho, Naka-Ku,**
**Yokohama-Shi, Kanagawa 231(JP)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 238 576 B1

## Beschreibung

Die Erfindung betrifft eine chirale getiltete smektische flüssigkristalline Phase mit ferroelektrischen Eigenschaften mit mindestens zwei flüssigkristallinen Verbindungen, dadurch gekennzeichnet, daß sie mindestens eine stickstoffhaltige heterocyclische Verbindung der Formel II,

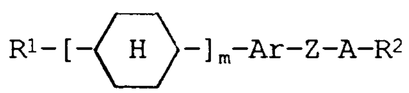 II

worin

$R^1$ und $R^2$ jeweils eine geradkettige Alkylgruppe mit 1-15 C-Atomen, worin auch eine oder mehrere $CH_2$-Gruppen durch -O-, -CO-O- oder -O-CO- ersetzt sein können, wobei zwei Heteroatome nicht direkt miteinander verknüpft sind, bedeutet,

oder einer der Reste $R^1$ und $R^2$ einen optisch aktiven Rest der Formel,

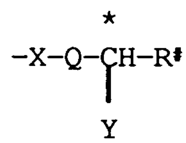

worin

- X -CO-O-, -O-CO-, -O-CO-O-, -CO-, -O-, -S-, -CH=CH-, -CH=CH-COO- oder eine Einfachbindung,
- Q Alkylen mit 1 bis 5 C-Atomen, worin auch eine nicht mit X verknüpfte $CH_2$-Gruppe durch -O-, -CO-, -O-CO-, -CO-O- oder -CH=CH- ersetzt sein kann, oder eine Einfachbindung,
- Y CN, Halogen, Methyl oder Methoxy, und
- $R^{\#}$ eine von Y verschiedene Alkylgruppe mit 1-18 C-Atomen,

bedeutet,

- m 0 oder 1,
- Ar eine der folgenden Gruppen

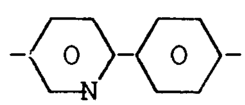 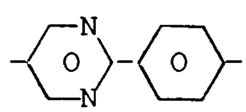

oder deren Spiegelbilder, bedeutet,

- Z -CO-O-, -O-CO-, $-CH_2O-$, $-OCH_2-$, $-CH_2CH_2-$ oder eine Einfachbindung

und

- A eine Gruppe der Formel

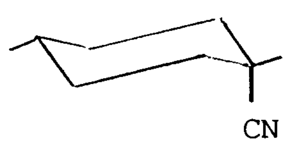

oder deren Spiegelbild

bedeutet, enthält.

Ähnliche Verbindungen sind in der WO-A 86 07 085, EP-A-107 759, EP-A-104 011, EP-A-111 695, EP-A-152 697 oder EP-A-152 808 beschrieben. Jedoch sind diese Verbindungen nicht als Komponenten smektischer flüssigkristalliner Phasen geeignet und/oder sie enthalten nicht das Strukturelement A und einen heterocyclischen, stickstoffhaltigen Ring.

Chirale getiltete smektische flüssigkristalline Phasen mit ferroelektrischen Eigenschaften können hergestellt werden, indem man Basis-Mischungen mit einer oder mehreren getilteten smektischen Phasen mit einem geeigneten chiralen Dotierstoff versetzt (L.A. Beresnev et al., Mol. Cryst. Liq. Cryst. 89, 327 (1982); H.R. Brand et al., J. Physique 44, (lett.), L-771 (1983).

Solche Phasen können als Dielektrika für schnell schaltende Displays verwendet werden, die auf dem von Clark und Lagerwall beschriebenen Prinzip der SSFLC-Technologie (N.A. Clark und S.T. Lagerwall, Appl. Phys. Lett. 36, 899 (1980); USP 4,367,924) auf der Basis der ferroelektrischen Eigenschaften der chiral getilteten Phase beruhen. In dieser Phase sind die langgestreckten Moleküle in Schichten angeordnet, wobei die Moleküle einen Tiltwinkel zur Schichtennormalen aufweisen. Beim Fortschreiten von Schicht zu Schicht ändert sich die Tiltrichtung um einen kleinen Winkel bezüglich einer senkrecht zu den Schichten stehenden Achse, so daß eine Helixstruktur ausgebildet wird. In Displays, die auf dem Prinzip der SSFLC-Technologie beruhen, sind die smektischen Schichten senkrecht zu den Platten der Zelle angeordnet. Die helixartige Anordnung der Tiltrichtungen der Moleküle wird durch einen sehr geringen Abstand der Platten (ca. 1-2 µm) unterdrückt. Dadurch werden die Längsachsen der Moleküle gezwungen, sich in einer Ebene parallel zu den Platten der Zelle anzuordnen, wodurch zwei ausgezeichnete Tiltorientierungen entstehen. Durch Anlegen eines geeigneten elektrischen Wechselfeldes kann in der eine spontane Polarisation aufweisenden flüssigkristalline Phase zwischen diesen beiden Zuständen hin- und hergeschaltet werden. Dieser Schaltvorgang ist wesentlich schneller als bei herkömmlichen verdrillten Zellen (TN-LCD's), die auf nematischen Flüssigkristallen basieren.

Ein großer Nachteil für viele Anwendungen der derzeit verfügbaren Materialien mit chiral getilteten smektischen Phasen (wie z.B. Sc*) ist, daß die dielektrische Anisotropie Werte größer Null oder, falls negativ, nur wenig von Null verschiedene Werte aufweist. Negative Werte der dielektrischen Anisotropie sind erforderlich, falls die erforderliche planare Orientierung durch Überlagerung des Ansteuerfeldes mit einem AC-Haltefeld mit kleiner Amplitude bewirkt wird (J.M. Geary, SID-Tagung, Orlando/Florida, April/Mai 1985, Vortrag 8.3). Der Einsatz von Materialien mit stark negativer dielektrischer Anisotropie führt meist zu einer starken Verminderung der Spontanpolarisation und/oder zu ungünstigen Werten für Pitch und/oder Tilt. Darüberhinaus wird meist der Temperaturbereich der ferroelektrischen Phasen in ungünstiger Weise eingeengt.

Es wurde nun gefunden, daß die Verwendung von Verbindungen der Formel II als Komponenten chiral getilteter smektischer Mischungen die erwähnten Nachteile wesentlich vermindern kann. Die Verbindungen der Formel II sind somit als Komponenten chiral getilteter smektischer flüssigkristalliner Phasen vorzüglich geeignet. Insbesondere sind mit ihrer Hilfe chemisch besonders stabile chiral getiltete smektische flüssigkristalline Phasen mit günstigen ferroelektrischen Phasenbereichen, insbesondere mit breiten Sc* Phasenbereichen, negativer dielektrischer Anisotropie, günstiger Pitchhöhe und für derartige Phasen hohen Werten für die spontane Polarisation herstellbar. P ist die spontane Polarisation in $nC/cm^2$.

Die Verbindungen der Formel II besitzen einen breiten Anwendungsbereich. In Abhängigkeit von der Auswahl der Substituenten können diese Verbindungen als Basismaterialien dienen, aus denen flüssigkristalline smektische Phasen zum überwiegenden Teil zusammengesetzt sind; es können aber auch Verbindungen der Formel II flüssigkristalline Basismaterialien aus anderen Verbindungsklassen zugesetzt werden, um beispielsweise die dielektrische und/oder optische Anisotropie und/oder die Viskosität und/oder die spontane Polarisation und/oder den Phasenbereich und/oder den Tiltwinkel und/oder den Pitch eines solchen Dielektrikums zu variieren. Gegenstand der Erfindung sind somit die Verbindungen der Formel II sowie deren Verwendung als Komponenten (chiraler getilteter) smektischer flüssigkristalliner Phasen. Gegenstand der Erfindung sind ferner smektische flüssigkristalline Phasen, insbesondere chirale getiltete smektische Phasen, mit einem Gehalt an mindestens einer Verbindung der Formel II sowie Flüssigkristallanzeigeelemente, insbesondere elektrooptische Anzeigeelemente, die derartige Phasen enthalten.

In den Verbindungen der vor- und nachstehenden Formeln bedeuten $R^1$ und $R^2$ eine geradkettige Alkylkette mit vorzugsweise 5 bis 12 C-Atomen, worin auch eine oder mehrere $CH_2$-Gruppen durch -O-, -CO-O- oder -O-CO- ersetzt sein können. Alkyl bedeutet beispielsweise bevorzugt Pentyl, Hexyl, Heptyl, Octyl, Nonyl, Decyl, Undecyl oder Dodecyl, ferner Methyl, Ethyl, Propyl, Butyl, 2-, 3- oder 4-Oxapentyl, 2-, 3-, 4-oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6-, oder 4,6-Dioxaheptyl, 1,4-Dioxaoctyl, 1,4,7-Trioxaoctyl, 1,4-Dioxanonyl, 1,4-Dioxadecyl.

Einer der Reste $R^1$ und $R^2$ kann auch ein optisch aktiver organischer Rest der Formel

$$-X-Q-\overset{*}{C}H(Y)-R^{\#}$$

sein,
worin

- X -CO-O-, -O-CO-, -O-CO-O-, -CO-, -O-, -S-, -CH = CH-, -CH = CH-COO- oder eine Einfachbindung,
- Q Alkylen mit 1 bis 5 C-Atomen, worin auch eine nicht mit X verknüpfte $CH_2$-Gruppe durch -O-, -CO-, -O-CO-, -CO-O- oder -CH = CH- ersetzt sein kann, oder eine Einfachbindung,
- Y CN, Halogen, Methyl oder Methoxy, und
- $R^{\#}$ eine von Y verschiedene Alkylgruppe mit 1 bis 18 C-Atomen,

bedeutet.

- X ist vorzugsweise -CO-O-, -O-CO-, -CH = CH-COO- (trans) oder eine Einfachbindung. Besonders bevorzugt sind -CO-O- und -O-CO-.
- Q ist vorzugsweise $-CH_2-$, $-CH_2CH_2-$ oder eine Einfachbindung, insbesondere bevorzugt eine Einfachbindung.
- Y ist vorzugsweie $CH_3$-, -CN oder Cl, insbesondere bevorzugt -CN.
- $R^{\#}$ ist vorzugsweise geradkettiges Alkyl mit 1 bis 10, insbesondere mit 1 bis 7 C-Atomen.

Unter den Verbindungen der Formel II sowie der vor- und nachstehenden Teilformeln sind diejenigen bevorzugt, in denen mindestens einer der darin enthaltenen Reste eine der angegebenen bevorzugten Bedeutungen hat.

Die Verbindungen der Formel II besitzen eine hohe chemische Stabilität. Sie sind farblos und gut mischbar mit allen gebräuchlichen Flüssigkristallen. Ihr Einsatz in flüssigkristallinen Phasen führt zu breiteren Mesophasenbereichen und verbesserten Werten für die Spontanpolarisation in chiral getilteten smektischen Phasen. Die erfindungsgemäßen Phasen eignen sich somit sehr gut für flüssigkristalline Phasen für Displays, die auf dem Prinzip der SSFLC-Technologie beruhen. Ferner eignen sie sich jedoch auch für andere elektrooptische Anzeigevorrichtungen, wie beispielsweise TN-Zellen oder Guest-Host-Zellen. Hier dienen sie neben der Erweiterung des Mesophasenbereichs insbesondere zur Einstellung von negativen Werten für die dielektrische Anisotropie und zur Verbesserung der elastischen Konstanten.

Die Verbindungen der Formel II können beispielsweise durch Umsetzung von entsprechenden Cyclohexancarbonitrilen (Formel II, $R^2$ = H) mit einem Halogenid der Formel $R^2$-Halogen erhalten werden.

Das Nitril wird zweckmäßig zunächst mit einer starken Base wie NaH, $NaNH_2$, Lithiumdiisopropylamid, -piperidid oder -2,5-diisopropylpiperidid oder K-tert.Butylat in das entsprechende Carbanion übergeführt, vorzugsweise in einem inerten Lösungsmittel, beispielsweise einem Kohlenwasserstoff wie Toluol, einem Ether wie THF oder Dioxan, einem Amid wie DMF, einem Sulfoxid wie Dimethylsulfoxid oder einem Gemisch derartiger Lösungsmittel. Nach Zugabe von $R^2$-Halogen hält man zweckmäßig 0,5 bis 16 Stunden bei Temperaturen zwischen 0° und 150°.

Die Halogenide sind beispielsweise aus den entsprechenden Alkoholen erhältlich. Halogen ist vorzugsweise Brom oder Jod.

Die erfindungsgemäßen Phasen enthalten vorzugsweise mindestens drei, insbesondere mindestens fünf Verbindungen der Formel II Besonders bevorzugt sind erfindungsgemäße chiral getiltete smektische flüssigkristalline Phasen, deren achirale Basismischung neben Verbindungen der Formel II mindestens eine andere Komponente mit negativer oder betragsmäßig kleiner positiver dielektrischer Anisotropie enthält. Diese weiteren Komponente(n) der chiralen Basismischung können 1 bis 50 %, vorzugsweise 10 bis 25 %, der Basismischung ausmachen. Als weitere Komponenten mit betragsmäßig kleiner positiver oder negativer dielektrischer Anisotropie eignen sich Verbindungen der Teilformeln Va bis Vp:

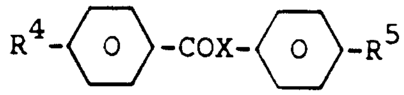

Va

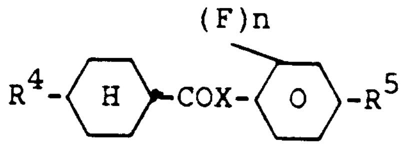

Vb

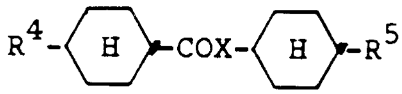

Vc

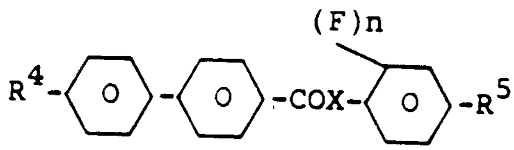

Vd

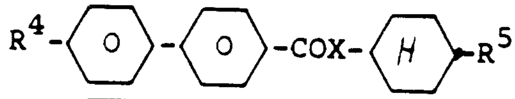

Ve

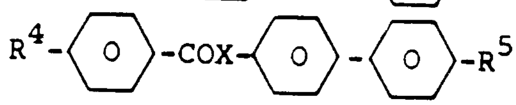

Vf

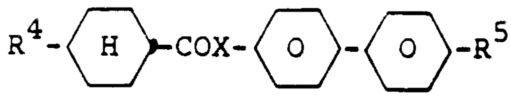

Vg

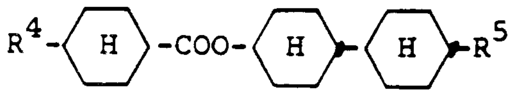

Vh

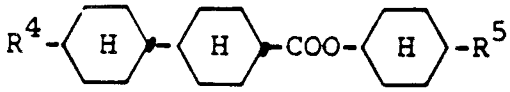

Vi

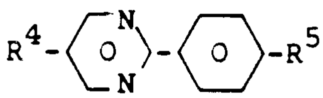

Vj

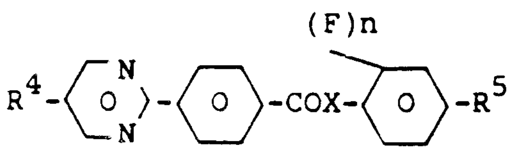

Vk

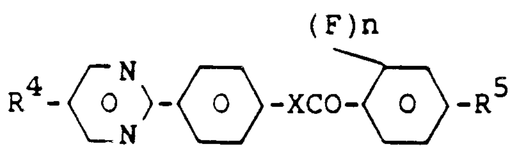

Vl

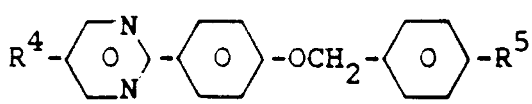

Vm

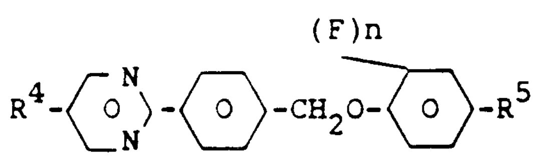

Vn

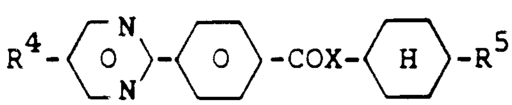

Vo

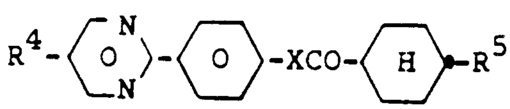

Vp

$R^4$ und $R^5$ sind jeweils vorzugsweise geradkettiges Alkyl, Alkoxy, Alkanoyloxy oder Alkoxycarbonyl mit jeweils 3 bis 12 C-Atomen. X ist vorzugsweise O. n ist 0 oder 1.

Besonders bevorzugt sind die Verbindungen der Teilformeln Va, Vb, Vd und Vf, worin $R^4$ und $R^5$ jeweils geradkettiges Alkyl oder Alkoxy mit jeweils 5 bis 10 C-Atomen bedeutet.

Die Verbindungen der Teilformeln Vc, Vh und Vi eignen sich als Zusätze zur Schmelzpunkterniedrigung und werden normalerweise den Basismischungen mit nicht mehr als 5 %, vorzugsweise 1 bis 3 %, zugesetzt. $R^4$ und $R^5$ bedeuten in den Verbindungen der Teilformeln Vc, Vh und Vi vorzugsweise geradkettiges Alkyl mit 2 bis 7, vorzugsweise 3 bis 5, C-Atomen. Eine weitere zur Schmelzpunktserniedrigung in den erfindungsgemäßen Phasen geeignete Verbindungsklasse ist diejenige der Formel

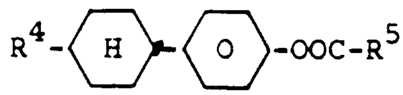

worin $R^4$ und $R^5$ die für Vc, Vh und Vi angegebene bevorzugte Bedeutung haben.

Als weitere Komponenten mit negativer dielektrischer Anisotropie eignen sich weiterhin Verbindungen enthaltend das Strukturelement B oder C.

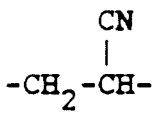

B

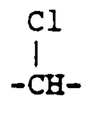

C

Bevorzugte Verbindungen dieser Art entsprechen den Formein VIb und VIc:

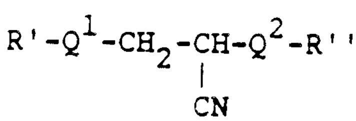

VIb

$R^1$-$Q^3$-$Q^4$-R'''     VIc

R' und R'' bedeuten jeweils vorzugsweise geradkettige Alkyl- oder Alkoxy-Gruppen mit jeweils 2 bis 10 C-Atomen. $Q^1$ und $Q^2$ bedeuten jeweils 1,4-Phenylen, trans1,4-Cyclohexylen, 4'4'-Biphenylyl, 4-(trans-4-Cyclohexyl)-phenyl, trans,trans-4,4'-Bicyclohexyl oder eine der Gruppen $Q^1$ und $Q^2$ auch eine Einfachbindung.

$Q^3$ und $Q^4$ bedeuten jeweils 1,4-Phenylen, 4,4'-Biphenylyl oder trans-1,4-Cyclohexylen. Eine der Gruppen $Q^3$ und $Q^4$ kann auch 1,4-Phenylen bedeuten, worin mindestens eine CH-Gruppe durch N ersetzt ist. R''' ist ein optisch aktiver Rest mit einem asymmetrischen Kohlenstoffatom der Struktur

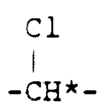

oder

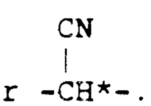

.

Besonders bevorzugte Verbindungen der Formel VIc sind diejenigen der Formel VIc':

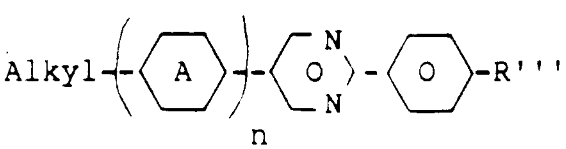

VIc'

worin A 1,4-Phenylen oder trans-1,4-Cyclohexylen und n 0 oder 1 bedeutet.

Die Herstellung der erfindungsgemäßen Phasen erfolgt in an sich üblicher Weise. In der Regel werden die Komponenten ineinander gelost, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Die folgenden Beispiele sollen die Erfindung erläutern. Vor- und nachstehend bedeuten Prozentangaben Gewichtsprozent; alle Temperaturen sind in Grad Celsius angegeben. Es bedeuten ferner: K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Beispiel 1

Zu 10,1 g Diisopropylamin in 70 ml THF werden unter Feuchtigkeitsausschluß und Stickstoffatmosphäre bei -10 °C nacheinander 62,5 ml einer 1,6 m Lösung von n-Butyllithium in Hexan und 36,1 g trans-4-[5-(p-n-Heptylphenyl)-2-pyrimidinyl]-cyclohexancarbonitril (EP-OS 00 14 885) in 50 ml THF getropft. Dann wird das Reaktionsgemisch ebenfalls bei -10 °c 20 Minuten gerührt. Anschließend gibt man 16,6 g 1-Brompentan zu und rührt dann 20 Minuten bei Raumtemperatur. Das Reaktionsgemisch wird wie üblich aufgearbeitet und das Produkt durch Chromatographie und Kristallisation gereinigt. Man erhält r-1-Cyan-1-n-pentyl-cis-4-[5-(p-n-heptylphenyl)-2-pyrimidinyl]-cyclohexan.

Analog werden hergestellt:

r-1-Cyan-1-propyl-cis-4-[5-(p-heptylphenyl)-2-pyrimidinyl]-cyclohexan
r-1-Cyan-1-butyl-cis-4-[5-(p-heptylphenyl)-2-pyrimidinyl]-cyclohexan
r-1-Cyan-1-hexyl-cis-4-[5-(p-heptylphenyl)-2-pyrimidinyl]-cyclohexan
r-1-Cyan-1-heptyl-cis-4-[5-(p-heptylphenyl)-2-pyrimidinyl]-cyclohexan
r-1-Cyan-1-octyl-cis-4-[5-(p-heptylphenyl)-2-pyrimidinyl]-cyclohexan
r-1-Cyan-1-nonyl-cis-4-[5-(p-heptylphenyl)-2-pyrimidinyl]-cyclohexan
r-1-Cyan-1-decyl-cis-4-[5-(p-heptylphenyl)-2-pyrimidinyl]-cyclohexan
r-1-Cyan-1-propyl-cis-4-[5-(p-octylphenyl)-2-pyrimidinyl]-cyclohexan
r-1-Cyan-1-butyl-cis-4-[5-(p-octylphenyl)-2-pyrimidinyl]-cyclohexan
r-1-Cyan-1-pentyl-cis-4-[5-(p-octylphenyl)-2-pyrimidinyl]-cyclohexan
r-1-Cyan-1-hexyl-cis-4-[5-(p-octylphenyl)-2-pyrimidinyl]-cyclohexan
r-1-Cyan-1-heptyl-cis-4-[5-(p-octylphenyl)-2-pyrimidinyl]-cyclohexan
r-1-Cyan-1-octyl-cis-4-[5-(p-octylphenyl)-2-pyrimidinyl]-cyclohexan
r-1-Cyan-1-nonyl-cis-4-[5-(p-octylphenyl)-2-pyrimidinyl]-cyclohexan
r-1-Cyan-1-decyl-cis-4-[5-(p-octylphenyl)-2-pyrimidinyl]-cyclohexan
r-1-Cyan-1-propyl-cis-4-[5-(p-nonylphenyl)-2-pyrimidinyl]-cyclohexan
r-1-Cyan-1-butyl-cis-4-[5-(p-nonylphenyl)-2-pyrimidinyl]-cyclohexan
r-1-Cyan-1-hexyl-cis-4-(5-(p-nonylphenyl)-2-pyrimidinyl]-cyclohexan
r-1-Cyan-1-heptyl-cis-4-[5-(p-nonylphenyl)-2-pyrimidinyl]-cyclohexan
r-1-Cyan-1-octyl-cis-4-[5-(p-nonylphenyl)-2-pyrimidinyl]-cyclohexan
r-1-Cyan-1-nonyl-cis-4-[5-(p-nonylphenyl)-2-pyrimidinyl]-cyclohexan
r-1-Cyan-1-decyl-cis-4-[5-(p-nonylphenyl)-2-pyrimidinyl]-cyclohexan
r-1-Cyan-1-propyl-cis-4-[5-(p-decylphenyl)-2-pyrimidinyl]-cyclohexan
r-1-Cyan-1-butyl-cis-4-[5-(p-decylphenyl)-2-pyrimidinyl]-cyclohexan
r-1-Cyan-1-hexyl-cis-4-[5-(p-decylphenyl)-2-pyrimidinyl]-cyclohexan
r-1-Cyan-1-heptyl-cis-4-[5-(p-decylphenyl)-2-pyrimidinyl]-cyclohexan
r-1-Cyan-1-octyl-cis-4-[5-(p-decylphenyl)-2-pyrimidinyl]-cyclohexan
r-1-Cyan-1-nonyl-cis-4-[5-(p-decylphenyl)-2-pyrimidinyl]-cyclohexan
r-1-Cyan-1-decyl-cis-4-[5-(p-decylphenyl)-2-pyrimidinyl]-cyclohexan

Beispiel 2

Zu einer Lösung von 4,6 g Natrium in 150 ml Methanol gibt man 34,7 g p-(4-n-Hexyl-4-cyanocyclohexyl)-benzamidinhydrochlorid und 20,4 g 2-n-Pentyl-3-ethoxyacrolein (analog A. Villiger, A. Boller, M. Schadt, Z.Naturforsch. 34b, 1535, 1979), rührt 12 Stunden unter Stickstoffatmosphäre, säuert mit 3N HCl an und arbeitet wie üblich auf. Man erhält r-1-Cyan-1-n-hexyl-cis-4-[p-(5-n-pentylpyrimidinyl-2)-phenyl]-cyclohexan.

Analog wurden hergestellt:

r-1-Cyan-1-hexyl-cis-4-[p-(5-butylpyrimidinyl-2)-phenyl]-cyclohexan,
r-1-Cyan-1-hexyl-cis-4-[p-(5-hexylpyrimidinyl-2)-phenyl]-cyclohexan,
r-1-Cyan-1-hexyl-cis-4-[p-(5-heptylpyrimidinyl-2)-phenyl]-cyclohexan,
r-1-Cyan-1-hexyl-cis-4-[p-(5-octylpyrimidinyl-2)-phenyl]-cyclohexan,
r-1-Cyan-1-hexyl-cis-4-[p-(5-nonylpyrimidinyl-2)-phenyl]-cyclohexan,
r-1-Cyan-1-pentyl-cis-4-[p-(5-butylpyrimidinyl-2)-phenyl]-cyclohexan,
r-1-Cyan-1-pentyl-cis-4-[p-(5-pentylpyrimidinyl-2)-phenyl]-cyclohexan,
r-1-Cyan-1-pentyl-cis-4-[p-(5-hexylpyrimidinyl-2)-phenyl]-cyclohexan,
r-1-Cyan-1-pentyl-cis-4-[p-(5-heptylpyrimidinyl-2)-phenyl]-cyclohexan,
r-1-Cyan-1-pentyl-cis-4-[p-(5-octylpyrimidinyl-2)-phenyl]-cyclohexan,
r-1-Cyan-1-pentyl-cis-4-[p-(5-nonylpyrimidinyl-2)-phenyl]-cyclohexan,
r-1-Cyan-1-pentyl-cis-4-[p-(5-decylpyrimidinyl-2)-phenyl]-cyclohexan,
r-1-Cyan-1-heptyl-cis-4-[p-(5-propylpyrimidinyl)-2)-phenyl]-cyclohexan, F. 94°, K. 143°
r-1-Cyan-1-heptyl-cis-4-[p-(5-butylpyrimidinyl-2)-phenyl]-cyclohexan,
r-1-Cyan-1-heptyl-cis-4-[p-(5-pentylpyrimidinyl-2)-phenyl]-cyclohexan,
r-1-Cyan-1-heptyl-cis-4-[p-(5-hexylpyrimidinyl-2)-phenyl]-cyclohexan,
r-1-Cyan-1-heptyl-cis-4-[p-(5-heptylpyrimidinyl-2)-phenyl]-cyclohexan, F. 101°, K. 126°
r-1-Cyan-1-heptyl-cis-4-[p-(5-octylpyrimidinyl-2)-phenyl]-cyclohexan,
r-1-Cyan-1-heptyl-cis-4-[p-(5-nonylpyrimidinyl-2)-phenyl]-cyclohexan,
r-1-Cyan-1-heptyl-cis-4-[p-(5-decylpyrimidinyl-2)-phenyl]-cyclohexan,
r-1-Cyan-1-octyl-cis-4-[p-(5-butylpyrimidinyl-2)-phenyl]-cyclohexan,
r-1-Cyan-1-octyl-cis-4-[p-(5-pentylpyrimidinyl-2)-phenyl]-cyclohexan,
r-1-Cyan-1-octyl-cis-4-[p-(5-hexylpyrimidinyl-2)-phenyl]-cyclohexan,
r-1-Cyan-1-octyl-cis-4-[p-(5-heptylpyrimidinyl-2)-phenyl]-cyclohexan,
r-1-Cyan-1-octyl-cis-4-(p-(5-octylpyrimidinyl-2)-phenyl]-cyclohexan,
r-1-Cyan-1-octyl-cis-4-[p-(5-nonylpyrimidinyl-2)-phenyl]-cyclohexan,
r-1-Cyan-1-octyl-cis-4-[p-(5-decylpyrimidinyl-2)-phenyl]-cyclohexan,
r-1-Cyan-1-nonyl-cis-4-[p-(5-butylpyrimidinyl-2)-phenyl]-cyclohexan,
r-1-Cyan-1-nonyl-cis-4-[p-(5-pentylpyrimidinyl-2)-phenyl]-cyclohexan,
r-1-Cyan-1-nonyl-cis-4-[p-(5-hexylpyrimidinyl-2)-phenyl]-cyclohexan,
r-1-Cyan-1-nonyl-cis-4-[p-(5-heptylpyrimidinyl-2)-phenyl]-cyclohexan,
r-1-Cyan-1-nonyl-cis-4-[p-(5-octylpyrimidinyl-2)-phenyl]-cyclohexan,
r-1-Cyan-1-nonyl-cis-4-[p-(5-nonylpyrimidinyl-2)-phenyl]-cyclohexan,
r-1-Cyan-1-nonyl-cis-4-[p-(5-decylpyrimidinyl-2)-phenyl]-cyclohexan,
r-1-Cyan-1-decyl-cis-4-[p-(5-butylpyrimidinyl-2)-phenyl]-cyclohexan,
r-1-Cyan-1-decyl-cis-4-[p-(5-pentylpyrimidinyl-2)-phenyl]-cyclohexan,
r-1-Cyan-1-decyl-cis-4-[p-(5-hexylpyrimidinyl-2)-phenyl]-cyclohexan,
r-1-Cyan-1-decyl-cis-4-[p-(5-heptylpyrimidinyl-2)-phenyl]-cyclohexan,
r-1-Cyan-1-decyl-cis-4-[p-(5-octylpyrimidinyl-2)-phenyl]-cyclohexan,
r-1-Cyan-1-decyl-cis-4-[p-(5-nonylpyrimidinyl-2)-phenyl]-cyclohexan,
r-1-Cyan-1-decyl-cis-4-[p-(5-decylpyrimidinyl-2)-phenyl]-cyclohexan,
r-1-Cyan-1-butyl-cis-4-[p-(5-butyl-pyrimidinyl-2)-phenyl]-cyclohexan,
r-1-Cyan-1-butyl-cis-4-[p-(5-pentyl-pyrimidinyl-2)-phenyl]-cyclohexan,
r-1-Cyan-1-butyl-cis-4-[p-(5-hexyl-pyrimidinyl-2)-phenyl]-cyclohexan,
r-1-Cyan-1-butyl-cis-4-[p-(5-heptyl-pyrimidinyl-2)-phenyl]-cyclohexan,
r-1-Cyan-1-butyl-cis-4-[p-(5-octyl-pyrimidinyl-2)-phenyl]-cyclohexan,
r-1-Cyan-1-butyl-cis-4-[p-(5-nonyl-pyrimidinyl-2)-phenyl]-cyclohexan,
r-1-Cyan-1-butyl-cis-4-[p-(5-decyl-pyrimidinyl-2)-phenyl]-cyclohexan,
r-1-Cyan-1-butyl-cis-4-[p-(5-(trans-4-butylcyclohexyl)-pyrimidinyl-2)-phenyl]-cyclohexan,
r-1-Cyan-1-butyl-cis-4-[p-(5-(trans-4-pentylcyclohexyl)-pyrimidinyl-2)-phenyl]-cyclohexan,
r-1-Cyan-1-butyl-cis-4-[p-(5-(trans-4-hexylcyclohexyl)-pyrimidinyl-2)-phenyl]-cyclohexan,
r-1-Cyan-1-butyl-cis-4-[p-(5-(trans-4-heptylcyclohexyl)-pyrimidinyl-2)-phenyl]-cyclohexan,
r-1-Cyan-1-butyl-cis-4-[p-(5-(trans-4-octylcyclohexyl)-pyrimidinyl-2)-phenyl]-cyclohexan,
r-1-Cyan-1-butyl-cis-4-[p-(5-(trans-4-nonylcyclohexyl)-pyrimidinyl-2)-phenyl]-cyclohexan,
r-1-Cyan-1-butyl-cis-4-[p-(5-(trans-4-decylcyclohexyl)-pyrimidinyl-2)-phenyl]-cyclohexan,
r-1-Cyan-1-pentyl-cis-4-[p-(5-(trans-4-butylcyclohexyl)-pyrimidinyl-2)-phenyl]-cyclohexan,
r-1-Cyan-1-pentyl-cis-4-[p-(5-(trans-4-pentylcyclohexyl)-pyrimidinyl-2)-phenyl]-cyclohexan,

r-1-Cyan-1-pentyl-cis-4-[p-(5-(trans-4-hexylcyclohexyl)-pyrimidinyl-2)-phenyl]-cyclohexan,
r-1-Cyan-1-pentyl-cis-4-[p-(5-(trans-4-heptylcyclohexyl)-pyrimidinyl-2)-phenyl]-cyclohexan,
r-1-Cyan-1-pentyl-cis-4-[p-(5-(trans-4-octylcyclohexyl)-pyrimidinyl-2)-phenyl]-cyclohexan,
r-1-Cyan-1-pentyl-cis-4-[P-(5-(trans-4-nonylcyclohexyl)-pyrimidinyl-2)-phenyl]-cyclohexan,
r-1-Cyan-1-pentyl-cis-4-[p-(5-(trans-4-decylcyclohexyl)-pyrimidinyl-2)-phenyl]-cyclohexan,
r-1-Cyan-1-hexyl-cis-4-[p-(5-(trans-4-butylcyclohexyl)-pyrimidinyl-2)-phenyl]-cyclohexan,
r-1-Cyan-1-hexyl-cis-4-[p-(5-(trans-4-pentylcyclohexyl)-pyrimidinyl-2)-phenyl]-cyclohexan,
r-1-Cyan-1-hexyl-cis-4-[p-(5-(trans-4-hexylcyclohexyl)-pyrimidinyl-2)-phenyl]-cyclohexan,
r-1-Cyan-1-hexyl-cis-4-[p-(5-(trans-4-heptylcyclohexyl)-pyrimidinyl-2)-phenyl]-cyclohexan,
r-1-Cyan-1-hexyl-cis-4-[p-(5-(trans-4-octylcyclohexyl)-pyrimidinyl-2)-phenyl]-cyclohexan,
r-1-Cyan-1-hexyl-cis-4-[p-(5-(trans-4-nonylcyclohexyl)-pyrimidinyl-2)-phenyl]-cyclohexan,
r-1-Cyan-1-hexyl-cis-4-[p-(5-(trans-4-decylcyclohexyl)-pyrimidinyl-2)-phenyl]-cyclohexan,
r-1-Cyan-1-heptyl-cis-4-[p-(5-(trans-4-butylcyclohexyl)-pyrimidinyl-2)-phenyl]-cyclohexan,
r-1-Cyan-1-heptyl-cis-4-[p-(5-(trans-4-pentylcyclohexyl)-pyrimidinyl-2)-phenyl]-cyclohexan,
r-1-Cyan-1-heptyl-cis-4-[p-(5-(trans-4-hexylcyclohexyl)-pyrimidinyl-2)-phenyl]-cyclohexan,
r-1-Cyan-1-heptyl-cis-4-[p-(5-(trans-4-heptylcyclohexyl)-pyrimidinyl-2)-phenyl]-cyclohexan,
r-1-Cyan-1-heptyl-cis-4-[p-(5-(trans-4-heptylcyclohexyl)-pyrimidinyl-2)-phenyl]-cyclohexan,
r-1-Cyan-1-heptyl-cis-4-[p-(5-(trans-4-nonylcyclohexyl)-pyrimidinyl-2)-phenyl]-cyclohexan,
r-1-Cyan-1-heptyl-cis-4-[p-(5-(trans-4-decylcyclohexyl)-pyrimidinyl-2)-phenyl]-cyclohexan,
r-1-Cyan-1-octyl-cis-4-[p-(5-(trans-4-butylcyclohexyl)-pyrimidinyl-2)-phenyl]-cyclohexan,
r-1-Cyan-1-octyl-cis-4-[p-(5-(trans-4-pentylcyclohexyl)-pyrimidinyl-2)-phenyl]-cyclohexan,
r-1-Cyan-1-octyl-cis-4-[p-(5-(trans-4-hexylcyclohexyl)-pyrimidinyl-2)-phenyl]-cyclohexan,
r-1-Cyan-1-octyl-cis-4-[p-(5-(trans-4-heptylcyclohexyl)-pyrimidinyl-2)-phenyl]-cyclohexan,
r-1-Cyan-1-octyl-cis-4-[p-(5-(trans-4-octylcyclohexyl)-pyrimidinyl-2)-phenyl]-cyclohexan,
r-1-Cyan-1-octyl-cis-4-[p-(5-(trans-4-nonylcyclohexyl)-pyrimidinyl-2)-phenyl]-cyclohexan,
r-1-Cyan-1-octyl-cis-4-[p-(5-(trans-4-decylcyclohexyl)-pyrimidinyl-2)-phenyl]-cyclohexan,
r-1-Cyan-1-nonyl-cis-4-[p-(5-(trans-4-butylcyclohexyl)-pyrimidinyl-2)-phenyl]-cyclohexan,
r-1-Cyan-1-nonyl-cis-4-[p-(5-(trans-4-pentylcyclohexyl)-pyrimidinyl-2)-phenyl]-cyclohexan,
r-1-Cyan-1-nonyl-cis-4-[p-(5-(trans-4-hexylcyclohexyl)-pyrimidinyl-2)-phenyl]-cyclohexan,
r-1-Cyan-1-nonyl-cis-4-[p-(5-(trans-4-heptylcyclohexyl)-pyrimidinyl-2)-phenyl]-cyclohexan,
r-1-Cyan-1-nonyl-cis-4-[p-(5-(trans-4-octylcyclohexyl)-pyrimidinyl-2)-phenyl]-cyclohexan,
r-1-Cyan-1-nonyl-cis-4-[p-(5-(trans-4-nonylcyclohexyl)-pyrimidinyl-2)-phenyl]-cyclohexan,
r-1-Cyan-1-nonyl-cis-4-[p-(5-(trans-4-decylcyclohexyl)-pyrimidinyl-2)-phenyl]-cyclohexan

Beispiel 3

Man stellt eine flüssigkristalline Phase her, bestehend aus

| | |
|---|---|
| 4 % | 2-p-Heptyloxyphenyl-5-heptylpyrimidin, |
| 30 % | 2-p-Undecyloxyphenyl-5-hexylpyrimidin, |
| 30 % | 2-p-Nonyloxyphenyl-5-nonylpyrimidin, |
| 12 % | r-1-Cyan-cis-4-[p-(5-heptylpyrimidinyl-2)-phenyl]-cyclohexan-1-carbonsäurepentylester, |
| 14 % | r-1-Cyan-cis-4-[p-(5-octylpyrimidinyl-2)-phenyl]-cyclohexan-1-carbonsäurebutylester und |
| 10 % | p-(5-Hexylpyrimidinyl-2)-phenyl-2-chlorpropionat (optisch aktiv). |

Beispiel 4

Man stellt eine flüssigkristalline Phase her, bestehend aus

| | |
|---|---|
| 20 % | 4-(5-Hexylpyrimidin-2-yl)-phenyl-(p-pentylbenzyl)-ether, |
| 20 % | 4-(5-Heptylpyrimidin-2-yl)-phenyl-(p-hexylbenzyl)-ether, |
| 20 % | 4-(5-Nonylpyrimidin-2-yl)-phenyl-(p-propylbenzyl)-ether, |
| 5 % | 4-(5-Nonylpyrimidin-2-yl)-phenyl-(p-cyanbenzyl)-ether, |
| 10 % | r-1-Cyan-cis-4-[p-(5-heptylpyrimidinyl-2)-phenyl]-cyclohexan-1-carbonsäurebutylester, |
| 15 % | r-1-Cyan-cis-4-[p-(5-octylpyrimidinyl-2)-phenyl]-cyclohexan-1-carbonsäurepentylester und |
| 10 % | p-(5-Hexylpyrimidinyl-2)-phenyl-2-chlorpropionat (optisch aktiv). |

Beispiel 5

Eine flüssigkristalline Phase bestehend aus

| | |
|---|---|
| 3 % | 2-p-Hexyloxyphenyl-5-heptylpyrimidin, |
| 3 % | 2-p-Heptyloxyphenyl-5-heptylpyrimidin, |
| 3 % | 2-p-Octyloxyphenyl-5-heptylpyrimidin, |
| 3 % | 2-p-Nonyloxyphenyl-5-heptylpyrimidin, |
| 8 % | 2-p-Hexyloxyphenyl-5-nonylpyrimidin, |
| 25 % | 2-p-Nonyloxyphenyl-5-nonylpyrimidin, |
| 30 % | r-1-Cyan-cis-4-(4'-octyloxybiphenyl-4-yl)-1-octylcyclohexan, |
| 15 % | r-1-Cyan-cis-4-(4'-nonanoyloxybiphenyl-4-yl)-1-butylcyclohexan und |
| 10 % | r-1-Cyan-cis-4-[p-(5-nonylpyrimidin-2-yl)-phenyl]-1-(2-methylbutyl)-cyclohexan (optisch aktiv) |

hat K -8° $S_C^x$ 65° $S_A^x$ und P = 8 $nC/cm^2$ bei 20°.

Beispiel 6

Man stellt eine flüssigkristalline Phase her bestehend aus

| | |
|---|---|
| 3 % | 2-p-Hexyloxyphenyl-5-heptylpyrimidin, |
| 3 % | 2-p-Heptyloxyphenyl-5-heptylpyrimidin, |
| 3 % | 2-p-Octyloxyphenyl-5-heptylpyrimidin, |
| 3 % | 2-p-Nonyloxyphenyl-5-heptylpyrimidin, |
| 8 % | 2-p-Hexyloxyphenyl-5-nonylpyrimidin, |
| 25 % | 2-p-Nonyloxyphenyl-5-nonylpyrimidin, |
| 20 % | r-1-Cyan-1-(trans-4-octylcyclohexyl-ethyl)-cis-4-(p-octyloxyphenyl)-cyclohexan, |
| 10 % | r-1-Cyan-1-(trans-4-octylcyclohexyl-ethyl)-cis-4-(p-heptyloxyphenyl)-cyclohexan, |
| 10 % | r-1-Cyan-1-(trans-4-octylcyclohexyl-ethyl)-cis-4-(p-octylphenyl)-cyclohexan, |
| 5 % | r-1-Cyan-1-(trans-4-octylcyclohexyl-ethyl)-cis-4-(p-heptylphenyl)-cyclohexan, |
| 10 % | r-1-Cyan-cis-4-[p-(5-octylpyrimidin-2-yl)-phenyl]-1-(2-methylbutyl)-cyclohexan (optisch aktiv). |

Beispiel 7

Eine flüssigkristalline Phase bestehend aus

| | |
|---|---|
| 3 % | 2-p-Hexyloxyphenyl-5-heptylpyrimidin, |
| 3 % | 2-p-Heptyloxyphenyl-5-heptylpyrimidin, |
| 3 % | 2-p-Octyloxyphenyl-5-heptylpyrimidin, |
| 3 % | 2-p-Nonyloxyphenyl-5-heptylpyrimidin, |
| 8 % | 2-p-Hexyloxyphenyl-5-nonylpyrimidin, |
| 25 % | 2-p-Nonyloxyphenyl-5-nonylpyrimidin, |
| 30 % | r-1-Cyan-cis-4-[p-(p-octyloxybenzoyloxy)-phenyl]-1-octylcyclohexan |
| 15 % | r-1-Cyan-cis-4-[p-(p-octyloxybenzoyloxy)-phenyl]-1-pentylcyclohexan |
| 10 % | r-1-Cyan-cis-4-[p-(5-nonyloxypyrimidin-2-yl)-phenyl]-1-(2-methylbutyl)-cyclohexan (otpisch aktiv) |

hat K -10° $S_C^x$ 61° $S_A^x$ und P = 10 nC/cm$^2$ bei 20°.

Beispiel 8

Man stellt eine flüssigkristalline Phase her bestehend aus

| | |
|---|---|
| 3 % | 2-p-Hexyloxyphenyl-5-heptylpyrimidin, |
| 3 % | 2-p-Heptyloxyphenyl-5-heptylpyrimidin, |
| 3 % | 2-p-Octyloxyphenyl-5-heptylpyrimidin, |
| 3 % | 2-p-Nonyloxyphenyl-5-heptylpyrimidin, |
| 8 % | 2-p-Hexyloxyphenyl-5-nonylpyrimidin, |
| 25 % | 2-p-Nonyloxyphenyl-5-nonylpyrimidin, |
| 15 % | 1-Cyan-1-trans-4-(p-heptyloxyphenyl)-cyclohexyl-2-(trans-4-octylcyclohexyl)-ethan, |
| 15 % | 1-Cyan-1-trans-4-(p-octyloxyphenyl)-cyclohexyl-2-(trans-4-octylcyclohexyl)-ethan, |
| 10 % | 1-Cyan-1-trans-4-(p-octylphenyl)-cyclohexyl-2-(trans-4-butylcyclohexyl)-ethan, |
| 5 % | 1-Cyan-1-trans-4-(p-heptylphenyl)-cyclohexyl-2-(trans-4-butylcyclohexyl)-ethan und |
| 10 % | r-1-Cyan-cis-4-[p-(5-octylpyrimidin-2-yl)-phenyl]-1-(2-methylbutyl)-cyclohexan. |

Beispiel 9

Man stellt eine flüssigkristalline Phase her bestehend aus

| | |
|---|---|
| 3 % | 2-p-Hexyloxyphenyl-5-heptylpyrimidin, |
| 3 % | 2-p-Heptyloxyphenyl-5-heptylpyrimidin, |
| 3 % | 2-p-Octyloxyphenyl-5-heptylpyrimidin, |
| 3 % | 2-p-Nonyloxyphenyl-5-heptylpyrimidin, |
| 8 % | 2-p-Hexyloxyphenyl-5-nonylpyrimidin, |
| 25 % | 2-p-Nonyloxyphenyl-5-nonylpyrimidin, |
| 10 % | r-1-Cyan-cis-4-(p-octyloxyphenyl)-1-octylcyclohexan, |
| 10 % | r-1-Cyan-cis-4-(4'-octyloxycarbonylbiphenyl-4-yl)-1-octylcyclohexan, |
| 5 % | r-1-Cyan-cis-4-(p-heptyloxybenzoyloxy)-1-nonylcyclohexan, |
| 10 % | r-1-Cyan-cis-4-[p-(p-octylphenyl)-benzoyloxy]-1-butylcyclohexan |
| 5 % | r-1-Cyan-cis-4-[p-(p-octyloxyphenyl)-benzoyloxy]-1-butylcyclohexan und |
| 15 % | r-1-Cyan-cis-4-[p-(5-nonylpyrimidin-2-yl)-phenoxycarbonyl]-1-(2-methylbutyl)-cyclohexan (optisch aktiv). |

Beispiel 10

Zu einem Gemisch von 3,2 g 2-p-Hydroxyphenyl-5-hexylpyrimidin, 3,15 g 4-Cyan-4-heptylcyclohexancarbonsäure [erhältlich aus 4-Cyancyclohexancarbonsäure durch Umsetzung mit n-Heptylbromid in Gegenwart von 2 Äquivalenten Lithiumdiisopropylamid] und 150 mg 4-N,N'-Dimethylaminopyridin in 150 ml Dichlormethan gibt man bei 5 - 10° unter Feuchtigkeitsausschluß 2,8 g Dicyclohexylcarbodiimid in 50 ml

Methylenchlorid, rührt eine Stunde bei Raumtemperatur, entfernt das ausgefallene Harnstoffderivat und arbeitet das Filtrat wie üblich auf. Man erhält r-1-Cyan-1-heptylcyclohexan-cis-4-carbonsäure-p-(5-hexylpyrimidin-2-yl)-phenylester.

Analog werden hergestellt:

r-1-Cyan-1-heptylcyclohexan-cis-4-carbonsäure-p-(5-propylpyrimidin-2-yl)-phenylester
r-1-Cyan-1-heptylcyclohexan-cis-4-carbonsäure-p-(5-butylpyrimidin-2-yl)-phenylester
r-1-Cyan-1-heptylcyclohexan-cis-4-carbonsäure-p-(5-pentylpyrimidin-2-yl)-phenylester
r-1-Cyan-1-heptylcyclohexan-cis-4-carbonsäure-p-(5-heptylpyrimidin-2-yl)-phenylester
r-1-Cyan-1-heptylcyclohexan-cis-4-carbonsäure-p-(5-octylpyrimidin-2-yl)-phenylester
r-1-Cyan-1-heptylcyclohexan-cis-4-carbonsäure-p-(5-nonylpyrimidin-2-yl)-phenylester
r-1-Cyan-1-heptylcyclohexan-cis-4-carbonsäure-p-(5-decylpyrimidin-2-yl)-phenylester
r-1-Cyan-1-octylcyclohexan-cis-4-carbonsäure-p-(5-propylpyrimidin-2-yl)-phenylester
r-1-Cyan-1-octylcyclohexan-cis-4-carbonsäure-p-(5-butylpyrimidin-2-yl)-phenylester
r-1-Cyan-1-octylcyclohexan-cis-4-carbonsäure-p-(5-pentylpyrimidin-2-yl)-phenylester
r-1-Cyan-1-octylcyclohexan-cis-4-carbonsäure-p-(5-hexylpyrimidin-2-yl)-phenylester
r-1-Cyan-1-octylcyclohexan-cis-4-carbonsäure-p-(5-heptylpyrimidin-2-yl)-phenylester
r-1-Cyan-1-octylcyclohexan-cis-4-carbonsäure-p-(5-octylpyrimidin-2-yl)-phenylester
r-1-Cyan-1-octylcyclohexan-cis-4-carbonsäure-p-(5-nonylpyrimidin-2-yl)-phenylester
r-1-Cyan-1-octylcyclohexan-cis-4-carbonsäure-p-(5-decylpyrimidin-2-yl)-phenylester
r-1-Cyan-1-nonylcyclohexan-cis-4-carbonsäure-p-(5-propylpyrimidin-2-yl)-phenylester
r-1-Cyan-1-nonylcyclohexan-cis-4-carbonsäure-p-(5-butylpyrimidin-2-yl)-phenylester
r-1-Cyan-1-nonylcyclohexan-cis-4-carbonsäure-p-(5-pentylpyrimidin-2-yl)-phenylester
r-1-Cyan-1-nonylcyclohexan-cis-4-carbonsäure-p-(5-hexylpyrimidin-2-yl)-phenylester
r-1-Cyan-1-nonylcyclohexan-cis-4-carbonsäure-p-(5-heptylpyrimidin-2-yl)-phenylester
r-1-Cyan-1-nonylcyclohexan-cis-4-carbonsäure-p-(5-octylpyrimidin-2-yl)-phenylester
r-1-Cyan-1-nonylcyclohexan-cis-4-carbonsäure-p-(5-nonylpyrimidin-2-yl)-phenylester
r-1-Cyan-1-nonylcyclohexan-cis-4-carbonsäure-p-(5-decylpyrimidin-2-yl)-phenylester
r-1 Cyan-1-heptylcyclohexan-cis-4-carbonsäure-p-(5-propylpyridin-2-yl)-phenylester
r-1-Cyan-1-heptylcyclohexan-cis-4-carbonsäure-p-(5-butylpyridin-2-yl)-phenylester
r-1-Cyan-1-heptylcyclohexan-cis-4-carbonsäure-p-(5-pentylpyridin-2-yl)-phenylester
r-1-Cyan-1-heptylcyclohexan-cis-4-carbonsäure-p-(5-hexylpyridin-2-yl)-phenylester
r-1-Cyan-1 heptylcyclohexan-cis-4-carbonsäure-p-(5-heptylpyridin-2-yl)-phenylester
r-1-Cyan-1-heptylcyclohexan-cis-4-carbonsäure-p-(5-octylpyridin-2-yl)-phenylester
r-1-Cyan-1-heptylcyclohexan-cis-4-carbonsäure-p-(5-nonylpyridin-2-yl)-phenylester
r-1-Cyan-1-heptylcyclohexan-cis-4-carbonsäure-p-(5-decylpyridin-2-yl)-phenylester
r-1-Cyan-1-heptylcyclohexan-cis-4-carbonsäure-p-(5-butoxypyridin-2-yl)-phenylester
r-1-Cyan-1-heptylcyclohexan-cis-4-carbonsäure-p-(5-pentoxypyridin-2-yl)-phenylester
r-1-Cyan-1-heptylcyclohexan-cis-4-carbonsäure-p-(5-hexoxypyridin-2-yl)-phenylester
r-1-Cyan-1-heptylcyclohexan-cis-4-carbonsäure-p-(5-heptoxypyridin-2-yl)-phenylester
r-1-Cyan-1-heptylcyclohexan-cis-4-carbonsäure-p-(5-octoxypyridin-2-yl)-phenylester
r-1-Cyan-1-heptylcyclohexan-cis-4-carbonsäure-p-(5-nonoxypyridin-2-yl)-phenylester
r-1-Cyan-1-heptylcyclohexan-cis-4-carbonsäure-p-(5-decoxypyridin-2-yl)-phenylester
r-1-Cyan-1-octylcyclohexan-cis-4-carbonsäure-p-(5-propylpyridin-2-yl)-phenylester
r-1-Cyan-1-octylcyclohexan-cis-4-carbonsäure-p-(5-butylpyridin-2-yl)-phenylester
r-1-Cyan-1-octylcyclohexan-cis-4-carbonsäure-p-(5-pentylpyridin-2-yl)-phenylester
r-1-Cyan-1-octylcyclohexan-cis-4-carbonsäure-p-(5-hexylpyridin-2-yl)-phenylester
r-1-Cyan-1-octylcyclohexan-cis-4-carbonsäure-p-(5-heptylpyridin-2-yl)-phenylester
r-1-Cyan-1-octylcyclohexan-cis-4-carbonsäure-p-(5-octylpyridin-2-yl)-phenylester
r-1-Cyan-1-octylcyclohexan-cis-4-carbonsäure-p-(5-nonylpyridin-2-yl)-phenylester
r-1-Cyan-1-octylcyclohexan-cis-4-carbonsäure-p-(5-decylpyridin-2-yl)-phenylester
r-1-Cyan-1-octylcyclohexan-cis-4-carbonsäure-p-(5-butoxypyridin-2-yl)-phenylester
r-1-Cyan-1-octylcyclohexan-cis-4-carbonsäure-p-(5-pentoxypyridin-2-yl)-phenylester
r-1-Cyan-1-octylcyclohexan-cis-4-carbonsäure-p-(5-hexoxypyridin-2-yl)-phenylester
r-1-Cyan-1-octylcyclohexan-cis-4-carbonsäure-p-(5-heptoxypyridin-2-yl)-phenylester
r-1-Cyan-1-octylcyclohexan-cis-4-carbonsäure-p-(5-octoxypyridin-2-yl)-phenylester
r-1-Cyan-1-octylcyclohexan-cis-4-carbonsäure-p-(5-nonoxypyridin-2-yl)-phenylester
r-1-Cyan-1-octylcyclohexan-cis-4-carbonsäure-p-(5-decoxypyridin-2-yl)-phenylester
r-1-Cyan-1-nonylcyclohexan-cis-4-carbonsäure-p-(5-propylpyridin-2-yl)-phenylester

r-1-Cyan-1-nonylcyclohexan-cis-4-carbonsäure-p-(5-butylpyridin-2-yl)-phenylester
r-1-Cyan-1-nonylcyclohexan-cis-4-carbonsäure-p-(5-pentylpyridin-2-yl)-phenylester
r-1-Cyan-1-nonylcyclohexan-cis-4-carbonsäure-p-(5-hexylpyridin-2-yl)-phenylester
r-1-Cyan-1-nonylcyclohexan-cis-4-carbonsäure-p-(5-heptylpyridin-2-yl)-phenylester
r-1-Cyan-1-nonylcyclohexan-cis-4-carbonsäure-p-(5-octylpyridin-2-yl)-phenylester
r-1-Cyan-1-nonylcyclohexan-cis-4-carbonsäure-p-(5-nonylpyridin-2-yl)-phenylester
r-1-Cyan-1-nonylcyclohexan-cis-4-carbonsäure-p-(5-decylpyridin-2-yl)-phenylester
r-1-Cyan-1-nonylcyclohexan-cis-4-carbonsäure-p-(5-butoxypyridin-2-yl)-phenylester
r-1-Cyan-1-nonylcyclohexan-cis-4-carbonsäure-p-(5-pentoxypyridin-2-yl)-phenylester
r-1-Cyan-1-nonylcyclohexan-cis-4-carbonsäure-p-(5-hexoxypyridin-2-yl)-phenylester
r-1-Cyan-1-nonylcyclohexan-cis-4-carbonsäure-p-(5-heptoxypyridin-2-yl)-phenylester
r-1-Cyan-1-nonylcyclohexan-cis-4-carbonsäure-p-(5-octoxypyridin-2-yl)-phenylester
r-1-Cyan-1-nonylcyclohexan-cis-4-carbonsäure-p-(5-nonoxypyridin-2-yl)-phenylester
r-1-Cyan-1-nonylcyclohexan-cis-4-carbonsäure-p-(5-decoxypyridin-2-yl)-phenylester
r-1-Cyan-cis-4-heptylcyclohexancarbonsäure-p-(5-propylpyrimidin-2-yl)-phenylester
r-1-Cyan-cis-4-heptylcyclohexancarbonsäure-p-(5-butylpyrimidin-2-yl)-phenylester
r-1-Cyan-cis-4-heptylcyclohexancarbonsäure-p-(5-pentylpyrimidin-2-yl)-phenylester
r-1-Cyan-cis-4-heptylcyclohexancarbonsäure-p-(5-hexylpyrimidin-2-yl)-phenylester
r-1-Cyan-cis-4-heptylcyclohexancarbonsäure-p-(5-heptylpyrimidin-2-yl)-phenylester
r-1-Cyan-cis-4-heptylcyclohexancarbonsäure-p-(5-octylpyrimidin-2-yl)-phenylester
r-1-Cyan-cis-4-heptylcyclohexancarbonsäure-p-(5-nonylpyrimidin-2-yl)-phenylester
r-1-Cyan-cis-4-heptylcyclohexancarbonsäure-p-(5-decylpyrimidin-2-yl)-phenylester
p-(5-Propylpyrimidin-2-yl)-benzoesäure-(4-cyan-4-heptylcyclohexyl)-ester
p-(5-Butylpyrimidin-2-yl)-benzoesäure-(4-cyan-4-heptylcyclohexyl)-ester
p-(5-Pentylpyrimidin-2-yl)-benzoesäure-(4-cyan-4-heptylcyclohexyl)-ester
p-(5-Hexylpyrimidin-2-yl)-benzoesäure-(4-cyan-4-heptylcyclohexyl)-ester
p-(5-Heptylpyrimidin-2-yl)-benzoesäure-(4-cyan-4-heptylcyclohexyl)-ester
p-(5-Octylpyrimidin-2-yl)-benzoesäure-(4-cyan-4-heptylcyclohexyl)-ester
p-(5-Nonylpyrimidin-2-yl)-benzoesäure-(4-cyan-4-heptylcyclohexyl)-ester
p-(5-Decylpyrimidin-2-yl)-benzoesäure-(4-cyan-4-heptylcyclohexyl)-ester
p-(5-Butoxypyrimidin-2-yl)-benzoesäure-(4-cyan-4-heptylcyclohexyl)-ester
p-(5-Pentoxypyrimidin-2-yl)-benzoesäure-(4-cyan-4-heptylcyclohexyl)-ester
p-(5-Hexoxypyrimidin-2-yl)-benzoesäure-(4-cyan-4-heptylcyclohexyl)-ester
p-(5-Heptoxypyrimidin-2-yl)-benzoesäure-(4-cyan-4-heptylcyclohexyl)-ester
p-(5-Octoxypyrimidin-2-yl)-benzoesäure-(4-cyan-4-heptylcyclohexyl)-ester
p-(5-Nonoxypyrimidin-2-yl)-benzoesäure-(4-cyan-4-heptylcyclohexyl)-ester
p-(5-Decoxypyrimidin-2-yl)-benzoesäure-(4-cyan-4-heptylcyclohexyl)-ester
p-(5-Propylpyridin-2-yl)-benzoesäure-(4-cyan-4-heptylcyclohexyl)-ester
p-(5-Butylpyridin-2-yl)-benzoesäure-(4-cyan-4-heptylcyclohexyl)-ester
p-(5-Pentylpyridin-2-yl)-benzoesäure-(4-cyan-4-heptylcyclohexyl)-ester
p-(5-Hexylpyridin-2-yl)-benzoesäure-(4-cyan-4-heptylcyclohexyl)-ester
p-(5-Heptylpyridin-2-yl)-benzoesäure-(4-cyan-4-heptylcyclohexyl)-ester
p-(5-Octylpyridin-2-yl)-benzoesäure-(4-cyan-4-heptylcyclohexyl)-ester
p-(5-Nonylpyridin-2-yl)-benzoesäure-(4-cyan-4-heptylcyclohexyl)-ester
p-(5-Decylpyridin-2-yl)-benzoesäure-(4-cyan-4-heptylcyclohexyl)-ester
p-(5-Butoxypyridin-2-yl)-benzoesäure-(4-cyan-4-heptylcyclohexyl)-ester
p-(5-Pentoxypyridin-2-yl)-benzoesäure-(4-cyan-4-heptylcyclohexyl)-ester
p-(5-Hexoxypyridin-2-yl)-benzoesäure-(4-cyan-4-heptylcyclohexyl)-ester
p-(5-Heptoxypyridin-2-yl)-benzoesäure-(4-cyan-4-heptylcyclohexyl)-ester
p-(5-Octoxypyridin-2-yl)-benzoesäure-(4-cyan-4-heptylcyclohexyl)-ester
p-(5-Nonoxypyridin-2-yl)-benzoesäure-(4-cyan-4-heptylcyclohexyl)-ester
p-(5-Decoxypyridin-2-yl)-benzoesäure-(4-cyan-4-heptylcyclohexyl)-ester

Beispiel 11

Ein Gemisch von 61,2 g 2-p-Hydroxyphenyl-5-heptylpyridin [erhältlich aus 2-p-Methoxyphenyl-5-heptylpyridin durch basische Etherspaltung mit Kaliumtert.-butylat in N-Methylpyrrolidon bei 150°], 35 g Kaliumcarbonat und 85 g 4-Cyan-4-heptylcyclohexylmethyljodid [erhältlich durch Reduktion von 4-Cyan-4-heptyl-

cyclohexancarbonsäureethylester mit $LiBH_4$, Überführung des Carbinols in das Mesylat und Umsetzung mit NaJ/Aceton nach Finkelstein] in 500 ml Dimethylformamid wird unter Stickstoffatmosphäre bei 110° 8 Stunden gerührt. Nach üblicher Aufarbeitung erhält man
r-1-Cyan-1-heptyl-cis-4-[p-(5-heptylpyridin-2-yl)-phenoxymethyl]-cyclohexan.

Analog werden hergestellt:

r-1-Cyan-1-octyl-cis-4-[p-(5-propylpyridin-2-yl)-phenoxymethyl]-cyclohexan
r-1-Cyan-1-octyl-cis-4-[p-(5-butylpyridin-2-yl)-phenoxymethyl]-cyclohexan
r-1-Cyan-1-octyl-cis-4-[p-(5-pentylpyridin-2-yl)-phenoxymethyl]-cyclohexan
r-1-Cyan-1-octyl-cis-4-[p-(5-hexylpyridin-2-yl)-phenoxymethyl]-cyclohexan
r-1-Cyan-1-octyl-cis-4-[p-(5-heptylpyridin-2-yl)-phenoxymethyl]-cyclohexan
r-1-Cyan-1-octyl-cis-4-[p-(5-octylpyridin-2-yl)-phenoxymethyl]-cyclohexan
r-1-Cyan-1-octyl-cis-4-[p-(5-nonylpyridin-2-yl)-phenoxymethyl]-cyclohexan
r-1-Cyan-1-octyl-cis-4-[p-(5-decylpyridin-2-yl)-phenoxymethyl]-cyclohexan
r-1-Cyan-1-heptyl-cis-4-[p-(5-propylpyrimidin-2-yl)-phenoxymethyl]-cyclohexan
r-1-Cyan-1-heptyl-cis-4-[p-(5-butylpyrimidin-2-yl)-phenoxymethyl]-cyclohexan
r-1-Cyan-1-heptyl-cis-4-[p-(5-pentylpyrimidin-2-yl)-phenoxymethyl]-cyclohexan
r-1-Cyan-1-heptyl-cis-4-[p-(5-hexylpyrimidin-2-yl)-phenoxymethyl]-cyclohexan
r-1-Cyan-1-heptyl-cis-4-[p-(5-heptylpyrimidin-2-yl)-phenoxymethyl]-cyclohexan
r-1-Cyan-1-heptyl-cis-4-[p-(5-octylpyrimidin-2-yl)-phenoxymethyl]-cyclohexan
r-1-Cyan-1-heptyl-cis-4-[p-(5-nonylpyrimidin-2-yl)-phenoxymethyl]-cyclohexan
r-1-Cyan-1-heptyl-cis-4-[p-(5-decylpyrimidin-2-yl)-phenoxymethyl]-cyclohexan
r-1-Cyan-1-octyl-cis-4-[p-(5-propylpyrimidin-2-yl)-phenoxymethyl]-cyclohexan
r-1-Cyan-1-octyl-cis-4-[p-(5-butylpyrimidin-2-yl)-phenoxymethyl]-cyclohexan
r-1-Cyan-1-octyl-cis-4-[p-(5-pentylpyrimidin-2-yl)-phenoxymethyl]-cyclohexan
r-1-Cyan-1-octyl-cis-4-[p-(5-hexylpyrimidin-2-yl)-phenoxymethyl]-cyclohexan
r-1-Cyan-1-octyl-cis-4-[p-(5-heptylpyrimidin-2-yl)-phenoxymethyl]-cyclohexan
r-1-Cyan-1-octyl-cis-4-[p-(5-octylpyrimidin-2-yl)-phenoxymethyl]-cyclohexan
r-1-Cyan-1-octyl-cis-4-[p-(5-nonylpyrimidin-2-yl)-phenoxymethyl]-cyclohexan
r-1-Cyan-1-octyl-cis-4-[p-(5-decylpyrimidin-2-yl)-phenoxymethyl]-cyclohexan

## Patentansprüche

1. Chirale getiltete smektische flüssigkristalline Phase mit ferroelektrischen Eigenschaften mit mindestens zwei flüssigkristallinen Verbindungen, dadurch gekennzeichnet, daß sie mindestens eine stickstoffhaltige heterocyclische Verbindung der Formel II,

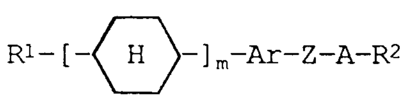

II

worin

$R^1$ und $R^2$ jeweils eine geradkettige Alkylgruppe mit 1-15 C-Atomen, worin auch eine oder mehrere $CH_2$-Gruppen durch -O-, -CO-O- oder -O-CO-ersetzt sein können, wobei zwei Heteroatome nicht direkt miteinander verknüpft sind, bedeutet,

oder einer der Reste $R^1$ und $R^2$ einen optisch aktiven Rest der Formel,

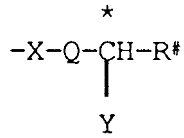

worin

X -CO-O-, -O-CO-, -O-CO-O-, -CO-, -O-, -S-, -CH=CH-, -CH=CH-COO- oder eine Einfachbindung,

Q Alkylen mit 1 bis 5 C-Atomen, worin auch eine nicht mit X verknüpfte $CH_2$-Gruppe durch -O-,

-CO-, -O-CO-, -CO-O- oder -CH=CH- ersetzt sein kann, oder eine Einfachbindung,

Y CN, Halogen, Methyl oder Methoxy, und

$R^{\#}$ eine von Y verschiedene Alkylgruppe mit 1-18 C-Atomen,

bedeutet,

m 0 oder 1,

Ar eine der folgenden Gruppen

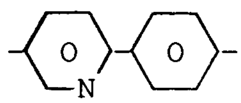

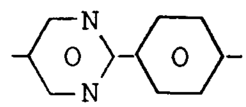

oder deren Spiegelbilder, bedeutet,

Z -CO-O-, -O-CO-, $-CH_2O-$, $-OCH_2-$, $-CH_2CH_2-$ oder eine Einfachbindung

und

A eine Gruppe der Formel

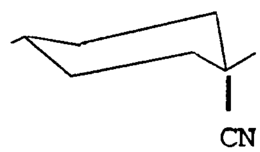

oder deren Spiegelbild

bedeutet,

enthält.

2. Phase nach Anspruch 1, dadurch gekennzeichnet, daß sie mindestens eine Verbindung der Formel II enthält, worin $R^1$ und $R^2$ geradkettiges Alkyl, Alkoxy, Alkanoyloxy, Alkoxycarbonyl, Oxaalkyl oder Alkoxycarbonyloxy mit jeweils 5-12 C-Atomen bedeuten.

3. Phase nach Anspruch 1, dadurch gekennzeichnet, daß sie mindestens eine Verbindung der Formel II enthält, worin $R^1$ und $R^2$ geradkettiges Alkyl oder Alkoxy mit jeweils 5 bis 12 C-Atomen bedeuten.

4. Phase nach Anspruch 1, dadurch gekennzeichnet, daß sie mindestens eine Verbindung der Formel II enthält, worin m = 0 ist.

5. Phase nach Anspruch 1, dadurch gekennzeichnet, daß sie mindestens eine Verbindung der Formel II enthält, worin einer der Reste $R^1$ und $R^2$ einen optisch aktiven organischen Rest der Formel

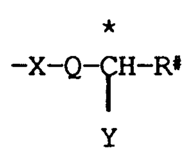

bedeutet.

6. Phase nach Anspruch 1, dadurch gekennzeichnet, daß sie mindestens eine Verbindung der Formel II enthält, worin Z -O-CO- oder -CO-O- bedeutet.

7. Elektrooptisches Anzeigeelement, dadurch gekennzeichnet, daß es als Dielektrikum eine Phase nach mindestens einem der Ansprüche 1-6 enthält.

## Claims

1. Chiral tilted smectic liquid-crystalline phase having ferroelectric properties and containing at least two liquid-crystalline compounds, characterized in that it contains at least one nitrogen-containing heterocyclic compound of the formula II

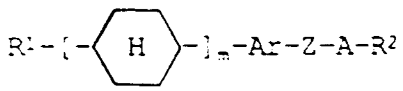

II

wherein

$R^1$ and $R^2$ are each a straight-chain alkyl group containing 1-15 C atoms, wherein one or more $CH_2$ groups may also be replaced by -O-, -CO-O- or -O-CO-, two heteroatoms not being directly linked to each other,

or one of the radicals $R^1$ and $R^2$ is an optically active radical of the formula

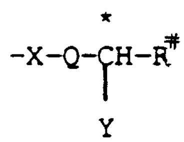

wherein

X is -CO-O-, -O-CO-, -O-CO-O-, -CO-, -O-, -S-, -CH = CH-, -CH = CH-COO- or a single bond,

Q is alkylene containing 1 to 5 C atoms, wherein a $CH_2$ group not linked to X may also be replaced by -O-, -CO-, -O-CO-, -CO-O- or -CH = CH-, or a single bond,

Y is CN, halogen, methyl or methoxy, and

$R^\#$ is an alkyl group different from Y and containing 1-18 C atoms,

m is 0 or 1,

Ar is one of the following groups

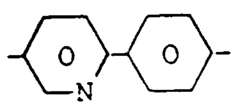

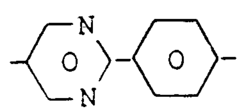

or their mirror images,

Z is -CO-O-, -O-CO-, $-CH_2O-$, $-OCH_2-$, $-CH_2CH_2-$or a single bond,

and

A is a group of the formula

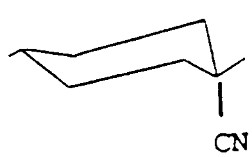

or its mirror image.

2. Phase according to Claim 1, characterised in that it contains at least one compound of the formula II, wherein $R^1$ and $R^2$ are straight-chain alkyl, alkoxy, alkanoyloxy, alkoxycarbonyl, oxaalkyl or alkoxycarbonyloxy, each containing 5-12 C atoms.

3. Phase according to Claim 1, characterized in that it contains at least one compound of the formula II, wherein $R^1$ and $R^2$ are straight-chain alkyl or alkoxy, each containing 5 to 12 C atoms.

4. Phase according to Claim 1, characterized in that it contains at least one compound of the formula II, wherein m = 0.

5. Phase according to Claim 1, characterized in that it contains at least one compound of the formula II, wherein one of the radicals $R^1$ and $R^2$ is an optically active organic radical of the formula

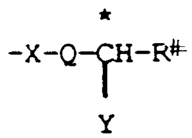

.

6. Phase according to Claim 1, characterized in that it contains at least one compound of the formula II, wherein Z is -O-CO- or -CO-O-.

7. Electrooptical display device, characterized in that it contains a phase according to at least one of Claims 1-6 as dielectric.

**Revendications**

1. Phase cristalline liquide smectique à inclinaison chirale ayant des propriétés ferro-électriques avec au moins deux composés à cristaux liquides, caractérisée en ce qu'elle contient au moins un composé hétérocyclique azoté de formule II

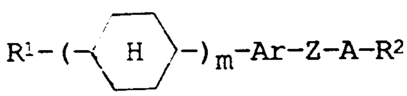

II

dans laquelle
$R^1$ et $R^2$ représentent chacun un groupe alcoyle à chaîne droite en $C_1$ à $C_{15}$, où également un ou plusieurs groupes $CH_2$ peut(peuvent) être remplacé(s) par -O-, -CO-O- ou -O-CO-, où deux hétéroatomes ne peuvent pas, être reliés directement entre eux,
ou l'un des radicaux $R^1$ et $R^2$ est un radical optiquement actif de formule

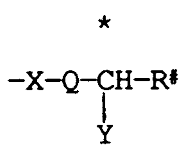

dans laquelle
X représente -CO-O, -O-CO-, -O-CO-O-, -CO-, -O-, -S-, -CH=CH-, -CH=CH-COO ou une liaison simple,
Q représente un alcoylène en $C_1$ à $C_5$, où également un groupe $CH_2$ non relié par X peut être remplacé par -O-, -CO-, -O-CO-, -CO-O- ou -CH=CH-, ou une liaison simple,
Y représente CH, un halogène, un méthyle ou un méthoxy, et
$R^{\#}$ représente un groupe alcoyle en $C_1$ à $C_{18}$ différent de Y,
m vaut 0 ou 1,
Ar représente l'un des groupes suivants

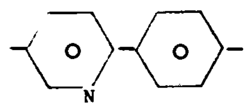

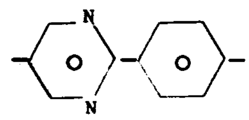

ou leur image spéculaire,
Z représente -CO-O-, -O-CO-, $-CH_2O-$, $-OCH_2-$, $-CH_2CH_2-$ ou une liaison simple
et
A représente un groupe de formule

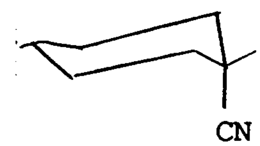

ou son image spéculaire.

2. Phase selon la revendication 1, caractérisée en ce qu'elle contient au moins un composé de formule II, dans laquelle $R^1$ et $R^2$ représentent un alcoyle, alcoxy, alcanoyloxy, alcoxycarbonyle, oxaalcoyle ou alcoxycarbonyloxy à chaîne droite avec à chaque fois de 5 à 12 atomes de carbone.

3. Phase selon la renvendication 1, caractérisée en ce qu'elle contient au moins un composé de formule II dans laquelle $R^1$ et $R^2$ représentent un alcoyle ou un alcoxy à chaîne droite avec à chaque fois de 5 à 12 atomes de carbone.

4. Phase selon la revendication 1, caractérisée en ce qu'elle contient au moins un composé de formule II dans laquelle m = 0.

5. Phase selon la revendication 1, caractérisée en ce qu'elle contient au moins un composé de formule II dans laquelle un des radicaux $R^1$ et $R^2$ représente un radical organique optiquement actif de formule

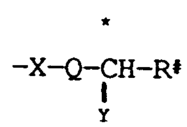

6. Phase selon la revendication 1, caractérisée en ce qu'elle contient au moins un composé de formule II dans laquelle Z représente -O-CO- ou -CO-O-.

7. Elément d'affichage électro-optique caractérisé en ce qu'il contient comme diélectrique une phase selon au moins une des revendications 1-6.